(19)

Europäisches
Patentamt

European
Patent Office

Office européen
des brevets

(11) **EP 3 925 447 A1**

(12) **EUROPEAN PATENT APPLICATION**

(43) Date of publication:
**22.12.2021 Bulletin 2021/51**

(21) Application number: **20305658.5**

(22) Date of filing: **16.06.2020**

(51) Int Cl.:
*A01N 59/16* (2006.01)          *A01N 25/10* (2006.01)
*A61K 9/16* (2006.01)          *A61K 33/38* (2006.01)
*A61L 2/238* (2006.01)          *A01P 1/00* (2006.01)
*A61K 47/36* (2006.01)          *B22F 9/24* (2006.01)
*B82Y 40/00* (2011.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Institut national de recherche pour l'agriculture
l'alimentation et l'environnement
75007 Paris (FR)**
• **Centre National de la Recherche Scientifique
75016 Paris (FR)**
• **Université Grenoble Alpes
38400 Saint Martin d'Hères (FR)**

• **Commissariat à l'Energie Atomique et aux
Energies
Alternatives (CEA)
75015 Paris (FR)**

(72) Inventors:
• **CAPRON, Isabelle
44300 Nantes (FR)**
• **MUSINO, Dafne
44000 Nantes (FR)**
• **RABILLOUD, Thierry
38320 Eybens (FR)**
• **LELONG, Cécile
38410 Saint Martin d'Uriage (FR)**
• **LUCHE, Sylvie
38500 Coublevie (FR)**

(74) Representative: **Ipside
6, Impasse Michel Labrousse
31100 Toulouse (FR)**

(54) **METHOD FOR PREPARING A HYBRID MATERIAL BASED ON UNMODIFIED
POLYSACCHARIDE NANOCRYSTALS AND SILVER NANOPARTICLES**

(57)     The invention relates to a method for preparing
a hybrid material containing polysaccharide nanocrystals
and silver nanoparticles, which comprises a step of preparing an aqueous reaction medium containing native
unmodified polysaccharide nanocrystals, a silver salt and
a silver ion-reducing agent, and a reaction step of allowing said reaction medium sufficient time to obtain therein
hybrid particles consisting of said polysaccharide nanoc-
rystals on the surface of which are grafted silver nano-
particles.

EP 3 925 447 A1

**Description**

The invention lies in the field of biocide materials.

More particularly, the invention relates to a method for preparing a hybrid material containing polysaccharide nanocrystals and silver nanoparticles. The invention also relates to a hybrid material obtainable by such a method, said material having particularly good biocidal properties, and to a biocide composition containing such a hybrid material. Another object of the invention is the use of this hybrid material or this composition as a biocide agent.

In the last decades, silver nanoparticles arose as one of the most efficient biocide agents, limiting and preventing micro-organisms and bacteria growing. Thanks to their high surface-to-volume ratio and their relatively low toxicity for human health, silver nanoparticles are widely employed in many fields, in particular in the fields of paints, cosmetics, dental materials, water treatment...

Even if the exact mechanism of action of silver nanoparticles on microorganisms is still not completely understood, it is known that their large surface area assures them a good contact with the microorganisms. It has been shown that the biocidal activity of silver nanoparticles is mainly related to the silver ions $Ag^+$ which can be released from these nanoparticles, this release being favored by the large surface area of the nanoparticles that ensures fast dissolution under favorable conditions. The released silver ions can then interact with components of the cell membrane, such as thiols, thereby inducing the microorganism inactivation.

It has been demonstrated by the prior art that several factors influence the biocidal activity of silver nanoparticles. This biocidal activity is size-dependent. Furthermore, it is crucial that the silver nanoparticles are in a well-dispersed state, avoiding aggregation which could lead to nanoparticle sedimentation and to a consequent decrease of the biocidal activity. It is also known that the oxidation state of the silver nanoparticles, i.e. the respective amounts of Ago and $Ag^+$ contained therein, could affect the biocidal effect since the release of $Ag^+$ ions is one of the main critical factors of the biocidal activity.

In many fields, for example in the field of systems used for surgical sutures, a long-term constant antimicrobial activity is required. In that case, silver nanoparticles are usually added in excess in the system. This approach leads to an excessive instantaneous release of silver, or to the persistence of unused silver nanoparticles, which can have a negative impact on the human health and the environment.

In order to address this issue, the prior art has sought solutions for making it possible to minimize the amount of silver nanoparticles used without detriment to the biocide effect.

In this context, it has been proposed by the prior art to develop biocide hybrid materials wherein silver nanoparticles are grafted at the surface of a bio-based substrate, thereby maximizing the silver nanoparticles specific surface and increasing the biocidal activity for a same given amount of silver nanoparticles. At the end-of-life of the material, the bio-degradation of the substrate can occur. This strategy has opened the road to more eco-friendly biocides. Natural polysaccharides such as cellulose, chitosan, starch, etc., have in particular been suggested as stabilizers for surface-mediated silver nanoparticles synthesis.

Being sustainable, low cost, low density, versatile via numerous possible interactions, with high specific surface, high modulus and anisotropic shape, nanocellulose has in particular drawn a large interest, for its potential to enable development of cost-effective materials with low carbon footprint, reducing reliance on fossil raw materials

Cellulose nanocrystals in particular represent an excellent bio-based support. Such nanocrystals can be extracted from several renewable natural sources such as plants, algae or bacterial cellulose, and they are well-dispersible in water, with a self-assemble ability. Several methods have been developed to synthesize hybrid materials containing silver nanoparticles anchored on the surface of cellulose nanocrystals. One of the most efficient methods is based on mixing nanocrystals with an aqueous solution of a silver precursor, and chemically reducing the silver ions contained therein. Nucleation and growth of silver nanoparticles then occur on the surface of the nanocrystals.

The publication of Uddin et al. (2014) Green Mater. 2, 183-192, describes an example of such a method for synthesizing silver nanoparticles, using TEMPO-oxidised cellulose nanocrystals as a starting material. This document discloses that carboxyl-functionalised cellulose nanocrystals obtained by TEMPO oxidation allow a considerable increase of interaction and stabilisation of the silver nanoparticles on the nanocrystals during the growth step.

Shi et al. (2015) J. Mater. Chem. B doi:10.1039/b000000x describes a method of synthesizing silver nanoparticles / cellulose nanocrystals hybrids, comprising a first step of modifying cellulose nanocrystals with dopamine, followed by *in-situ* generation and anchoring of silver nanoparticles on the surface of the nanocrystals through reduction of silver ions in the presence of ammonia and dopamine hydrochloride.

The publication of Drogat et al. (2011) J. Nanoparticle Res. 13, 1557-1562 describes a method of generating silver nanoparticles on cellulose nanocrystals, comprising a first step of periodate-oxidation of the cellulose nanocrystals, then contacting said surface-modified nanocrystals with a silver ion in the presence of ammonia.

All of these methods require many steps to be implemented, and are quite complex to perform. Moreover, the biocidal properties of the hybrid materials obtained thereby are not completely satisfying.

The present invention aims to overcome the drawbacks of the methods for preparing hybrid materials based on silver nanoparticles and nanocrystals of cellulose, and more generally nanocrystals of a polysaccharide, proposed by the prior art, in particular the drawbacks described above, by proposing a method for preparing such a hybrid material having very good biocidal properties, while containing a low amount of silver, this method being simple and cheap to carry out, and implementing as few steps as possible and as few reactants as possible.

It has now been discovered by the inventors that these objectives can be met, provided that some specific operating parameters are applied. In particular, the inventors have discovered that surprisingly, and contrarily to what is disclosed by the prior art, a hybrid material containing a very low amount of silver and having particularly good biocidal properties can be obtained using unmodified polysaccharide nanocrystals as the starting material which is brought into contact with a precursor of silver nanoparticles, this very simply and in very few steps.

Therefore, it is proposed by the invention a method for preparing a hybrid material in particulate form, containing polysaccharide nanocrystals and silver nanoparticles. Said method comprises:

- a step of preparing an aqueous reaction medium containing, and preferably consisting of, native unmodified nanocrystals of a polysaccharide, a silver salt and a silver ion-reducing agent, and wherein the molar ratio "reducing agent / silver salt" is between 1 and 2000 and the mass ratio "silver salt / polysaccharide nanocrystals" is between 0.0002 and 0.5;

- then a reaction step of allowing said reaction medium sufficient time to obtain therein hybrid particles comprising polysaccharide nanocrystals on the surface of which are grafted silver nanoparticles, forming the hybrid material of the invention.

During the reaction step, which may be qualified as a surface-mediated silver nanoparticles synthesis, silver nanoparticles are formed by chemical reduction, by the reducing agent, of the silver ions from the silver salt, and nucleation and growth on the surface of the unmodified polysaccharide nanocrystals, more particularly on the surface-hydroxyl groups thereof.

The formation of silver nanoparticles on the surface of the polysaccharide nanocrystals occurs very quickly when the silver-ion reducing agent is mixed with the polysaccharide nanocrystals and the silver salt, i.e. in a few seconds or minutes. It is within the skills of the person skilled in the art to determine the duration of the reaction step which is necessary to ensure that the desired hybrid particles are obtained therein. For this purpose, the person skilled in the art can monitor the color of the reaction medium, a change of color from translucent to yellow or dark yellow being indicative of the formation of silver nanoparticles, forming part of the hybrid material of the invention, in the reaction medium. The person skilled in the art can otherwise proceed to UV-visible spectroscopy analysis of the reaction medium at different times during the reaction step, the appearance of a peak at 400 nm, characteristic of small well-dispersed silver nanoparticles, being indicative of the formation of such nanoparticles on the surface of the polysaccharide nanocrystals.

Scanning Transmission Electron Microscopy then allows checking that all the silver nanoparticles are well-anchored on the surface of the polysaccharide nanocrystals, and that no "free" silver nanoparticles are present in the reaction medium.

In preferred embodiments, the method of the invention is devoid of any treatment step after the reaction step. By treatment step, it is meant herein any step which causes a modification of the structure of the hybrid particles, in particular of the polysaccharide nanocrystals or of the silver nanoparticles anchored to their surface. In particular, the method of the invention does preferably not comprise, after the reaction step, any step of oxidoreduction, for example using hydrogen peroxide, microwave treatment, etc.

Washing / purification steps, such as dialysis, are not considered as treatment steps in the meaning of the invention, as they do not cause any modification of the chemical structure of the hybrid particles. Besides, in particular embodiments of the invention, the method can optionally comprise a final step of purifying the hybrid material obtained at the end of the reaction step, in particular by dialysis, in order to eliminate any unreacted species left in the reaction medium.

By nanoparticles, it is meant here, generally speaking, particles at least one dimension of which is smaller than 100 nm. The silver nanoparticles of the hybrid material prepared by the method of the invention are advantageously such that all their dimensions are comprised between 3 and 20 nm.

The term "nanocrystal" herein means a crystal at least one dimension of which is comprised between 10 and 1000 nm, and preferably all dimensions of which are comprised between 10 and 1000 nm. The average size of polysaccharide nanocrystals varies depending on the source thereof. The average length of cellulose nanocrystals is for example from 150 to 200 nm when the cellulose originates from wood, whereas it can be close to 1000 nm for bacterial cellulose nanocrystals.

In preferred embodiments of the invention the polysaccharide nanocrystals, in particular the cellulose nanocrystals, have an elongated shape, and preferably an acicular shape, with a length of between 25 nm and 1000 nm and a width of between 5 and 30 nm.

The polysaccharide nanocrystals, in particular the cellulose nanocrystals, used in the method of the invention may have at least one, preferably several and more preferably all, of the following features:

- a length between 100 nm and 1000 nm, and a width between 5 and 20 nm;

- a length / width ratio greater than 1 and less than 100, and preferably between 10 and 55;

- a surface charge density of 0.6 e.nm$^{-2}$ or less, wherein e corresponds to an elementary charge.

The polysaccharide nanocrystals, in particular the cellulose nanocrystals, may comprise a charged surface, in particular a negatively charged surface, their surface charge density then being preferably comprised between 0.01 e.nm$^{-2}$ and 0.6 e.nm$^{-2}$. They may otherwise have a neutral surface, their surface charge density then being less than or equal to 0.01 e.nm$^{-2}$.

Nanocrystals differ in particular from nanofibrils, which are in a semi-crystalline state and which are obtained by mechanical treatment, whereas nanocrystals are obtained by chemical treatment, for example by acid hydrolysis, and have a mostly crystalline form.

"Native unmodified polysaccharide nanocrystals", "native polysaccharide nanocrystals" and "unmodified polysaccharide nanocrystals" are all employed herein to mean, in a conventional way, native nanocrystals as they are obtained at the end of the method implemented for their preparation, and which are unmodified, i.e. nanocrystals which have not been subjected to any subsequent treatment likely to modify the chemical structure of the surface thereof, and in particular to realize the substitution of the surface hydroxyl groups of the polysaccharide, and which have not been subjected to any step of surface coating with a polymer. For example, the polysaccharide nanocrystals incorporated in the reaction medium according to the invention have not been subjected beforehand to any surface-modification treatment such as TEMPO oxidation, periodate oxidation, modification with dopamine, polymer coating, etc.

Native polysaccharide nanocrystals can be prepared by any chemical treatment technique that is conventional *per se.* According to the preparation method used, the nanocrystals obtained can bear functional groups on their surface, such as sulfate or phosphate groups. No subsequent step of surface-modification of the polysaccharide groups exposed at the surface of the nanocrystals, nor any other modification of the chemical structure of the surface of the nanocrystals, is then performed before the implementation of the method of the invention for preparing a hybrid material based on polysaccharide nanocrystals and silver nanoparticles.

The method of the invention is therefore simpler, cheaper and quicker to implement than the methods of the prior art, which require a preliminary step of surface modification of the polysaccharide nanocrystals, and sometimes also a final step of modification of the hybrid material / hybrid particles obtained at the end of the reaction step involving polysaccharide nanocrystals, silver and a reducing agent. The method of the invention does nevertheless make it possible to obtain a hybrid material having very good long-term biocidal properties, with a very low amount of silver.

By biocidal property, it is meant here, in a conventional way, the ability to repel, render harmless or kill harmful organisms / microorganisms, in particular microbes such as viruses, bacteria, fungi, algae and lichens. The hybrid material prepared according to the invention has very-good long-term bactericide, virucide, fungicide, sporicide, algicide, etc., properties.

In particular, a minimal inhibitory concentration (MIC) as low as 16 $\mu$g of silver nanoparticles/ml, equivalent to 0.048 $\mu$g of silver, can be obtained with respect to microorganisms of the species *Bacillus subtilis.*

It will not be presumed here of the mechanisms responsible for such a high biocidal performance of the hybrid material obtained by the method of the invention. It can be assumed that the specific combination of operating parameters of the method of the invention results in the obtention of optimal size, shape, oxidation state and amount of well-dispersed silver nanoparticles nucleated on the surface of the polysaccharide nanocrystals, as well as a particularly good control of the release of silver ions Ag$^+$ over time.

Indeed, it has been discovered by the inventors that the method of the invention produces, grafted on the surface of the polysaccharide nanocrystals, silver nanoparticles which are spherical, very small, more particularly with a diameter between 3 and 20 nm, and an average diameter of 11 nm with a low polydispersity index, and which are very well distributed and dispersed on the surface of the nanocrystals. The specific surface of the silver nanoparticles is maximized, and the weight content of silver nanoparticles in the hybrid material can advantageously be controlled, preferably at a value less than 25 % by weight. Moreover, the hybrid material contains a high amount of the Ag$^+$ fraction, with respect to the Ago fraction, and therefore a high Ag$^+$/Ag$_0$ ratio, which promotes its biocidal activity, especially at contents of silver nanoparticles in the hybrid material of between 10 and 25 % by weight.

The high biocidal performance of the hybrid material prepared by the method of the invention is advantageously achieved without the need to use external stabilizing agents such as trisodium citrate or polyvinylpyrrolidone. Although such stabilizing agents may be added to the aqueous reaction medium in the first step of the method of the invention, in preferred embodiments of the invention, no such stabilizing agents are used.

The method of the invention makes it possible to prepare a hybrid material composed of a bio-sourced substrate grafted in surface with a low content of silver nanoparticles and a high long-lasting biocidal activity, with the lowest minimal inhibitory concentration ever reported in the literature. It is highly environmentally-friendly.

The method according to the invention may further respond to one or more of the features described below, implemented individually or in each of their technically operating combinations.

The molar ratio "reducing agent / silver salt" in the reaction medium is preferably between 1.5 and 68, and/or the mass ratio "silver salt / polysaccharide nanocrystals" in the reaction medium is preferably between 0.006 and 0.3. These ranges of values make it possible to obtain the best results in terms of biocidal activity, for a given amount of silver in the hybrid material obtained by the method of the invention.

The polysaccharide may be chosen from cellulose, chitin, starch, mannan and any of the mixtures thereof. The method of the invention may use, as starting material, a single type of polysaccharide nanocrystals or a mixture of different types of polysaccharide nanocrystals, for example a mixture of cellulose nanocrystals and chitin nanocrystals.

Cellulose is a particularly preferred polysaccharide in the context of the invention, as it allows for a particularly good dispersion of silver nanoparticles on the nanocrystals, and as cellulose nanocrystals can easily be prepared on an industrial scale.

Cellulose nanocrystals consist of parallel hydrogen-bonded cellulose chains, that are often obtained by acid hydrolysis of wood fibers. As an example, cellulose nanocrystals may be obtained from bleached Kraft pulp by acid hydrolysis, in particular with sulfuric acid, then neutralization to sodium form and spray drying. Methods for obtaining cellulose nanocrystals are for example described in the publication of Moon et al. (2011) Chem. Soc. Rev., 40, 3941-3994 or in the publication of Reid et al. (2017) Langmuir 33 (7), 1583-1598.

The nanocrystals thus obtained are qualified as native unmodified cellulose nanocrystals, in accordance with the invention. Such native cellulose nanocrystals, stabilized by surface negative charges, can be well-dispersed in aqueous vehicles and they can be used as a starting material in the method of the invention, without being subjected beforehand to any preliminary surface-modification step.

It is within the skills of the person skilled in the art to determine which compounds are reducing agents for the silver ions, and may be implemented in the method of the invention; mild reducing agents, in particular with a redox potential between 0.0 and -3.1 V, are particularly preferred. Preferably, the silver ion-reducing agent is chosen to be active at least at pH values between 5.5 and 6.5.

The reducing agent used in the method of the invention preferably has a high water-solubility. It is preferably chosen so as to be easy to manipulate and environmentally-friendly.

The reducing agent is preferably chosen from sodium borohydride $NaBH_4$, hydrazine, "green" reducing agents of plant origin, dimethylformamide, ammonia $NH_4OH$, ascorbic acid, etc. The method of the invention may implement a single silver-ion reducing agent or a plurality of such agents.

$NaBH_4$ is particularly preferred in the context of the invention, for its efficiency for reducing silver ions in aqueous solution. Moreover, the monomeric silver nanoparticles formed in the reaction step of a method of the invention using sodium borohydride as the reducing agent contain, at least initially, surface-absorbed borohydride ions, which increase the nanoparticles' dispersion capacity.

Any silver salt may be used in the method of the invention. Preferably, the silver salt is silver nitrate $AgNO_3$, silver chloride $AgCl$ or any of the mixtures thereof. The method of the invention may implement a single silver salt or a plurality of such salts

The mass ratio "silver / polysaccharide" in the reaction medium is preferably between 0.001 and 0.5, preferably between 0.004 and 0.2.

In particular embodiments of the invention, the concentration of the silver salt in the reaction medium is between 0.005 and 1 %, preferably between 0.013 and 0.6 %, by weight of silver salt with respect to the total volume of the reaction medium.

The ionic strength of the reaction medium is preferably set at a value less than 0.5 mol.$l^{-1}$. Such a feature advantageously limits the aggregation of the polysaccharide nanocrystals in the reaction medium, and favors a good dispersion of silver grafting on the surface thereof.

In particular embodiments of the invention, the step of preparing the aqueous reaction medium comprises the successive steps of mixing an aqueous suspension of the polysaccharide nanocrystals with the silver salt, in particular with an aqueous solution containing the silver salt, then, after a few seconds of stirring, in particular after 30 seconds to 2 minutes of stirring, for example after 1 minute of stirring, adding the reducing agent in the mixture. An advantage of such a method is that only mechanical stirring is necessary for the reaction to occur. No heating of the reaction medium is needed and no organic, and potentially dangerous, solvent has to be used. The cleaning steps are therefore also advantageously simplified, and the method of the invention is low energy-consuming.

In particular embodiments of the invention, the reaction step of the method is carried out according to one of the following features, preferably according to several, and more preferably to all, of these features:

- at a temperature between 18 and 25°C;

- and/or for at least 5 minutes, preferably for at least 10 minutes. Reaction times of least 30 minutes, and preferably between 1 and 24 hours, are particularly preferred as they promote a good dispersion of the hybrid particles in the reaction medium;

- and/or under stirring;

- and/or in the dark.

According to the polysaccharide used in the method of the invention, the reaction step may comprise a step of maintaining the pH of the reaction medium in a given range. By way of example, when the polysaccharide is chitin, the pH of the reaction medium is preferably controlled to be at a value of less than 6. When the polysaccharide is cellulose, the pH of the reaction medium is preferably not controlled, which advantageously simplifies the implementation of the method.

The invention also relates to a hybrid material obtainable, and in particular obtained, by a method according to the invention. This hybrid material consists of hybrid particles comprising unmodified polysaccharide nanocrystals on the surface of which are grafted silver nanoparticles. The silver nanoparticles have a spherical, or quasi-spherical, shape, and a diameter of between 3 and 20 nm, preferably with an average diameter of 11 nm. The hybrid particles of the invention contain 0.1 to 40 %, preferably 0.4 to 25 %, by weight of silver with respect to the total weight of said hybrid particles.

The silver nanoparticles are advantageously well-dispersed on the surface of the polysaccharide nanocrystals.

The polysaccharide may be cellulose, chitin, starch or mannan.

Furthermore, the weight ratio "silver / polysaccharide nanocrystals", in particular "silver / cellulose nanocrystals", is preferably between 0.1 and 50 %, preferably between 0.4 and 35 %.

The hybrid material of the invention is advantageously bio-based and safe to release in the environment.

It might be in a solid form, or dispersed in an aqueous vehicle.

The silver content thereof can advantageously be optimized according to the desired biocidal effect.

The hybrid material of the invention may have, in particular when the polysaccharide is cellulose, a minimal inhibitory concentration (MIC) against microorganisms of the species *Bacillus subtilis* as low as 16 $\mu$g of silver nanoparticles/ml. This minimal inhibitory concentration can be determined by a disk diffusion test after 48 hours of culture of the microorganisms at 30 °C on a solid culture medium, in the presence of the hybrid material.

Such a MIC is equivalent to a minimum weight amount of silver for obtaining a biocide effect of 0.048 $\mu$g.

Another object of the invention is a liquid biocide composition containing a hybrid material according to the invention in an aqueous vehicle, in particular in suspension in said vehicle.

In a particular embodiment of the invention, the biocide composition contains from 0.25 to 50 % by weight, preferably from 0.74 to 34.4 % by weight, of silver with respect to the total volume of the biocide composition.

The composition of the invention may contain any additive(s) that are conventional *per se* in the field of biocide compositions, for example surfactant and/or dispersing agent(s).

It may also contain additional active ingredient(s), such as additional antimicrobial agent(s), or any other active agent.

This hybrid material of the invention may be used in any field wherein a biocidal / antimicrobial effect might be of interest.

A further aspect of the invention is therefore the non-therapeutic use of a hybrid material according to the invention or of a biocide composition according to the invention, containing such a hybrid material, as a biocide agent.

The hybrid material and the biocide composition according to the invention may in particular be used for the protection of surfaces, more particularly of inert surfaces, against harmful organisms. The hybrid material of the invention may for example be incorporated in paints, or applied on a canvas / cloth in order to form a coating thereon or to impregnate it. Said application may be performed by any conventional technique, for example by spraying, dipping, etc.

The hybrid material and the biocide composition of the invention may in particular be used in the manufacture of packaging articles for food and/or cosmetic products. The hybrid material may in particular be integrated in a packaging article such as a film or paper / cardboard article for food packaging, or a plastic container for the packaging of a cosmetic product. The hybrid material of the invention may otherwise be applied, in solid form or in suspension in a liquid composition, so as to form a coating on a substate such as a paper or cardboard substrate or a polymer film, for example a polypropylene or polyethylene film. The applying of the hybrid material of the invention on the surface of a substrate, in particular of a polymer film, may be carried out in any way that is conventional *per se,* for example by spraying a biocide composition according to the invention on the surface of the substrate, then drying the substrate in order to obtain a solid coating on the surface thereof. The substrate may have been subjected to any adequate surface-modifying treatment beforehand. As an example, a polymer film may be surface-functionalized with a layer of cationic polyelectrolyte before the applying of the hybrid material of the invention, in order to promote the attachment of the hybrid particles on the surface of the film.

Another object of the invention is therefore a packaging article for the food industry or the cosmetic industry, containing a hybrid material according to the invention, in particular a polymer film coated with a hybrid material according to the invention.

The invention also relates to a method for assaying the long-term biocide activity of a material. This method comprises contacting the material to be tested, carried on a solid support, with a plate uniformly covered by the target organism, and incubating the assembly at an adequate temperature for growing said organism, then measuring the diameter of the organism growth-inhibition zone on the plate after a given long period of time, for example after 168 hours.

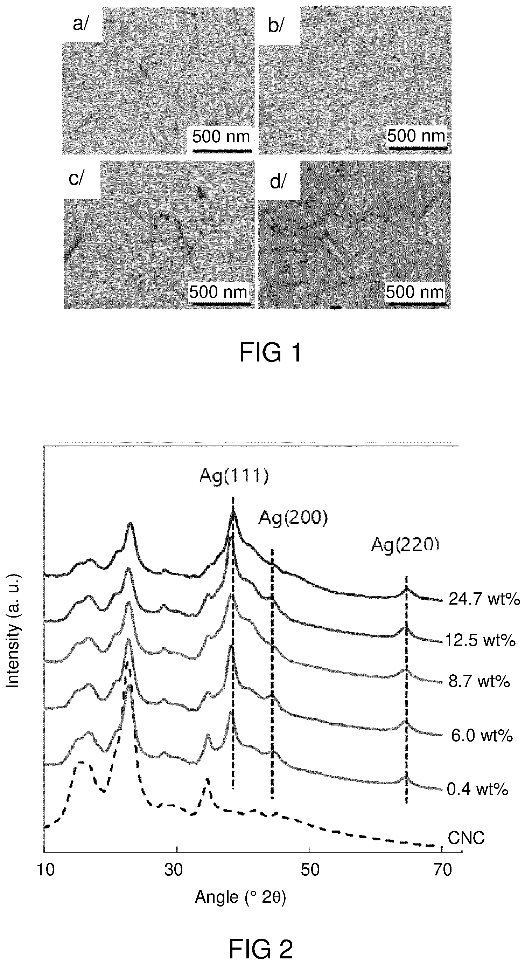

The features and advantages of the invention will emerge more clearly in the light of the following examples of implementation, provided for illustrative purposes only and in no way limitative of the invention, with the support of figures 1 to 6, in which:

- figure 1 shows scanning transmission electron microscopy images of hybrid particles prepared by a method according to the invention, based on unmodified cellulose nanocrystals and silver nanoparticles, comprising silver nanoparticles in a weight concentration of a/ 3 %, b/ 6 %, c/ 12.5 %, d/ 24.7 %;

- figure 2 shows X-ray powder diffraction diffractograms of, respectively, from the bottom to the top, native unmodified cellulose nanocrystals (CNC), and hybrid materials based on these cellulose nanocrystals and silver nanoparticles obtained by a method according to the invention, containing different weight concentrations of silver (0.4 wt%, 6.0 wt%, 8.7 wt%, 12.5 wt%, 24.7 wt%);

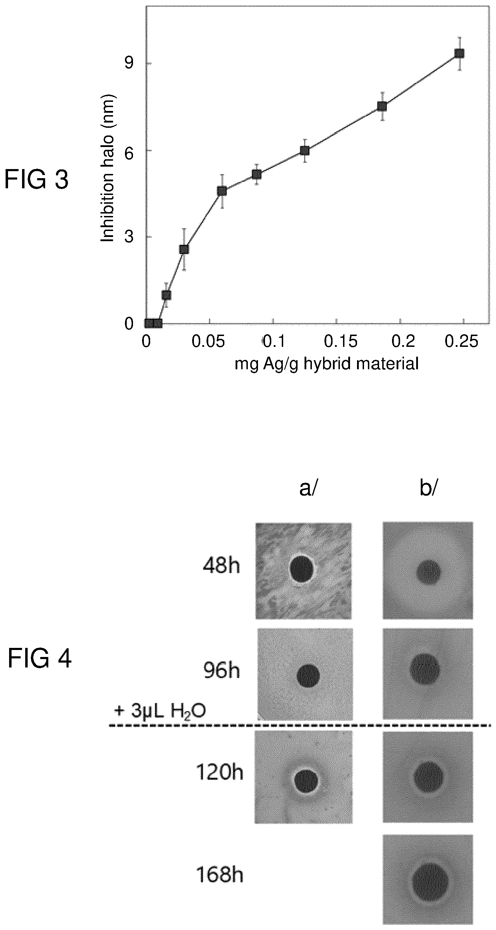

- figure 3 shows a graph representing the size of the inhibition halo after 48 h of incubation as a function of the silver content of an aqueous dispersion of hybrid particles based on native unmodified cellulose nanocrystals and silver nanoparticles prepared according to different embodiments of a method of the invention (implementing different concentrations of silver salt), in an assay for evaluating the biocide activity of these particles with respect to *Bacillus subtilis*;

- figure 4 shows optical microscopy images of inhibition halos obtained for hybrid particles based on native unmodified cellulose nanocrystals and silver nanoparticles prepared according to different embodiments of a method of the invention, containing respectively 8.7 wt% (a/) and 24.7 wt% (b/) of silver nanoparticles, after various incubation times (48h, 96h, 120h, 168h) in an assay for evaluating the biocide activity of these particles with respect to *Bacillus subtilis*;

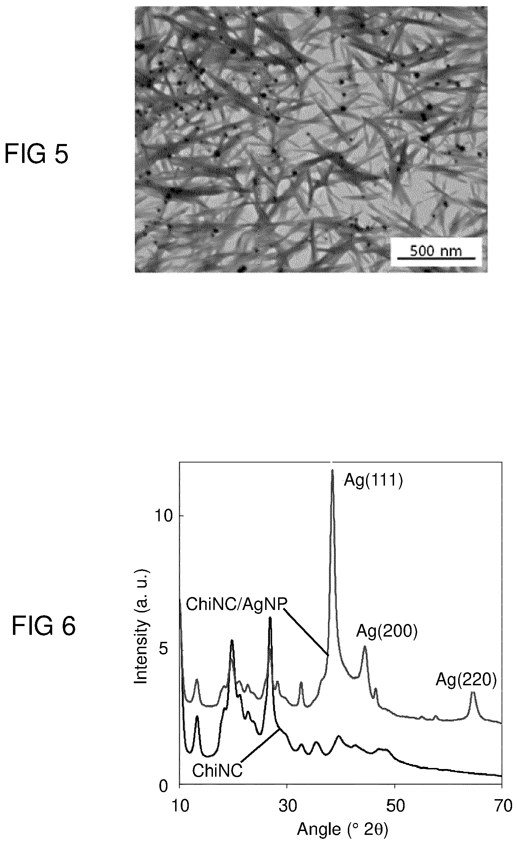

- figure 5 shows a scanning transmission electron microscopy image of hybrid particles prepared by a method according to the invention, based on chitin nanocrystals and silver nanoparticles at 23.5 wt%;

- and figure 6 shows X-ray powder diffraction diffractograms of, respectively, native chitin nanocrystals (ChiNC), and a hybrid material based on these chitin nanocrystals and silver nanoparticles obtained by a method according to the invention (ChiNC/AgNP).

**EXAMPLE 1** - Cellulose

**Materials and methods**

Materials

Native unmodified cellulose nano-crystals (CNCs) were purchased from CelluForce (product num. 2015-009). They were obtained from bleached Kraft pulp by acid hydrolysis and then neutralized to sodium form and spray dried (length = 183 $\pm$ 88 nm; cross-section = 6 $\pm$ 2 nm; aspect ratio = 31). Silver nitrate (AgNO$_3$, $\geq$99%), sodium borohydride (NaBH$_4$, $\geq$96%), were bought from Sigma-Aldrich and used without further purification. All the aqueous suspensions and solutions were prepared using ultra-pure water.

Synthesis of hybrid materials according to the invention

10 mL of an aqueous suspension of CNCs (2 g/L) was dialyzed against water for 3 days and then mixed at room temperature for 1 min with various amounts of AgNO$_3$ aqueous solution (50 mM, 15, 30, 60, 110, 160, 240, 330,

550 or 700 $\mu$L). Then, 500 $\mu$L of freshly-prepared $NaBH_4$ aqueous solution (100 mM) were added to reduce the $Ag^+$ ions, in order to obtain silver nanoparticles (AgNPs). $NaBH_4$ aqueous solution was put in ice to minimize its decomposition.

The final suspension was covered by aluminum foil in order to prevent AgNP oxidation by light and it was stirred at room temperature for 24 h.

The final suspension was dialyzed against water for 24 h.

Characterization

A Mettler-Toledo UV7 spectrophotometer equipped with a 10 mm quartz cell was used to record the light-visible absorbance of hybrid suspensions in the 300 - 900 nm range. All the samples were diluted (1:10) and ultra-pure water was used as a blank reference.

The AgNP content in aqueous suspensions of the hybrid particles was determined by digesting 1 mL of sample by 40 mL water/aqua regia mixture (i.e., 30%v aqua regia, $HCl/HNO_3$:3/1) and then analyzing it by atomic absorption spectroscopy (AAS) (ICE 3300 AAS, Thermo Fisher). A calibration curve was obtained using a silver standard solution (1000 $\mu$g/mL, Chem-Lab NV) at different concentrations, from 0.25 to 10 ppm. Two independent measurements were repeated for each sample. The final AgNP content was expressed in mg_AgNP/g_sample, wt%).

To perform scanning transmission electron microscopy (STEM) acquisitions, hybrid suspensions were water-diluted at 0.5 g/L in CNC content. 10 $\mu$L were deposited onto glow-discharged carbon coated grids (200 meshes, Delta Microscopies, France) for 2 min and the excess was removed using Whatman® filter paper. The grids were dried overnight in air and then coated with platinum layer by an ion-sputter coater (thickness = 0.5 nm). Images were recorded working with Quattro Scanning electron microscope (Thermo Scientific®) at 10kV using a STEM detector. The acquired images were analyzed by ImageJ software to determine the AgNP Feret's diameter (i.e., the largest distance of two tangents to the contour of the measured particle). For each sample, the size of at least 100 silver nanoparticles was evaluated to obtain an average value.

X-ray absorption near edge structure (XANES) spectra were recorded in transmission mode at the Ag K-edge (from 25250 to 27750 eV) on SAMBA beamline at synchrotron SOLEIL (Saint Aubin, France). Energy calibration of the Si (220) monochromator was calibrated to 25515.6 eV at the first inflection point of the Ag foil XANES spectrum. The freeze-dried hybrid suspensions were pressed to obtain circular pellets with diameter of 6 mm with a controlled amount of AgNPs to obtain an absorption edge jump close to 1. The pellets were put on a sample rod and immersed in a liquid nitrogen bath before being introduced into the He cryostat (T=20 K). Silver foil (Agfoil) and $AgNO_3$ aqueous solution with 1wt% glycerol ($AgH_2O$) were used as standards. For each sample, one scan was collected and then normalized and background-subtracted using software package Athena. XANES data were analyzed by a linear combination fitting procedure (LCF) using the fit range [Eo - 20 eV, Eo + 50 eV], with Eo set to 25514 eV and using Agfoil and $AgH_2O$ standards as fitting components. All the components weights were forced to be positive and the relative proportions of the components were forced to add up to 100%.

A Bruker D8 Discover diffractometer was used to record X-ray powder diffraction (XRD) diffractograms (10 min of acquisition). Cu-K$\alpha$1 radiation (Cu K$\alpha$1,1.5405 Å) was produced in a sealed tube at 40 kV and 40 mA, parallelized using a Gobël mirror parallel optic system and then collimated to produce a 500 mm beam diameter. The data were collected in a 2$\theta$ angle range from 3° to 70°. The AgNP crystallite size (CS) was determined using the Scherrer's equation:

$$CS = \frac{K\lambda}{\beta cos\theta}$$

where K is the shape factor (0.9), $\lambda$ is the X-ray wavelength (1.54 Å), $\beta$ is the full-width at half-maximum (FWHM) and $\theta$ is the angle of the diffraction peak of the crystalline phase (Bragg's angle). The FWHM was determined considering the AgNP characteristic peak at 2$\theta$ = 38°.

Conductometric measurements were performed to determine $Ag^+$ release in the aqueous hybrid suspensions. The conductivity of 10 mL of each dialyzed sample were monitored at different times (i.e., 48 h, 96 h, 120 h, 168 h at 21°C) using a Metrohm 856 Conductivity Module and it was recorded by Tiamo® Titration Software. The silver ions concentration was deduced using a calibration curve which related conductivity and $[Ag^+]$ and which was obtained by the analysis of various amount of a silver standard solution (1000 $\mu$g/mL, Chem-Lab NV) diluted in a 2 g/L CNC suspension ($[Ag^+]$ from 0 to 2.4 mM).

Biocide test - disk diffusion test

For the disk diffusion test, a wild-type *Bacillus subtilis* strain was used (*Bacillus subtilis subsp. Subtilis* (trpC(NCIB3610)), available under ID 1A1282 at the Bacillus Genetic Stock Center (BGSC)). The cells were grown in LB medium: 10 g/l tryptone, 5 g/l yeast extract and 5 g/l NaCl. A *Bacillus subtilis* culture grown overnight on liquid LB at 37°C was diluted to $A_{600nm}$= 0.1 in 10 ml of fresh LB medium and incubated at 37°C and 200 rpm until the $A_{600nm}$ reached the exponential phase ($\approx$ 0.6). 300 $\mu$l of this culture (or $10^8$ cells) were applied uniformly on the surface of a LB agar plate (22 ml LB-agar) before placing the paper disks (Blotting paper, grade 703 from VWR) of uniform size (5 mm) on the plate. For each suspension of hybrid particles, 3 $\mu$l were immediately deposited on the paper disks. Ultra-pure water and ethanol 70% were used as negative control and positive control, respectively. After 48 h at 30°C, the average diameter of the inhibition zone ("inhibition halo") was measured.

Biocide test - persistence test

For the biocidal activity persistence test, the biocide-impregnated paper disks from the above experiment were recovered, layered on a fresh bacteria plate exactly as described above and rehydrated with 3 $\mu$l of water. The plates were grown at 30°C for variable time periods (96 h, 120 h or 168 h), and the average diameter of the inhibition zones ("inhibition halos") was measured. All experiments were performed in triplicate (three independent growth cultures) with at least two technical replicates.

**Results**

Characterization of synthesized hybrid materials

CNC/AgNP hybrid materials were produced according to the invention, by addition of various amounts of aqueous solution of the silver precursor $AgNO_3$ at 50 mM, to a suspension of unmodified CNC in water. The reduction of silver ions by $NaBH_4$ led to an immediate color change of the suspension from translucent to yellow, indicating the formation of AgNPs. The formation of well-dispersed AgNPs derived from aggregation of monomeric Ag particles obtained by a reduction to zero valence Ag atoms and it was confirmed by the presence of a dominant in-plane absorption peak at $\lambda_{max}\sim$ 400 nm in the UV-Vis spectra, whose intensity increased with the AgNP content in the suspension.

In all the hybrid materials, the AgNPs obtained on the surface of the CNCs were well-defined spherical nanoparticles.

The AgNP content of the hybrids as determined by atomic absorption spectroscopy revealed a nominal AgNP content from 0.4 wt% to 24.7 wt% (mg AgNP/g sample, wt%). STEM images performed from dry CNC/AgNP hybrids, shown in Figure 1 for 4 examples of AgNP content in the hybrid material, clearly show very well-dispersed AgNPs attached onto CNCs surface, for all the synthesized hybrid materials. The average AgNP diameter measured from microscopic images is around 10 nm for all the hybrid materials, with a value of 11 nm at 3.0 wt% and 24.7 wt%.

After the AgNP formation, characteristic silver peaks appeared in the XRD patterns of all the CNC/AgNPs hybrid suspensions, as shown in figure 2. A peak at 38.1°, usually associated to the (111) lattice plane of face-centered cubic (fcc) silver (JCPDS Card No. 89-3722), was detected. Other characteristic silver peaks with weaker intensity were detected at 44.1° and 64.2°, corresponding to (200) and (220) crystalline planes, thus confirming the isotropic nature of the crystals and the average crystallite size (CS) was estimated equal to 3.3 $\pm$ 0.3 nm.

The characteristics of the hybrid material (CNC/AgNP hybrid particles) obtained are indicated in table 1. These characteristics include the AgNP oxidation state (more particularly the % of the Ago oxidation state, with respect to the total amount of Ag), determined from XANES spectra.

Table 1 - Characteristics of hybrid materials prepared according to the invention - [1]measured by AAS, [2]measured by STEM, [3]measured by XRD, [4]measured by XANES

| Volume of $AgNO_3$ sol. (50 mM) in the reaction medium ($\mu$l) | $NaBH_4$/ $AgNO_3$ molar ratio | AgNP content in the hybrid material (mg/g, wt%)[1] | Average Feret's diameter (nm)[2] | Crystallite size (nm)[3] | Ago (%)[4] |
|---|---|---|---|---|---|
| 15 | 67.7 | 0.4 $\pm$ 0.03 | 11 $\pm$ 5 | 3.5 | - |
| 30 | 33.4 | 0.9 $\pm$ 0.04 | - | - | - |
| 60 | 16.7 | 1.6 $\pm$ 0.02 | - | - | - |
| 110 | 9.1 | 3.0 $\pm$ 0.03 | 11 $\pm$ 8 | - | 91 |
| 160 | 6.3 | 6.0$\pm$0.01 | 10 $\pm$ 7 | 3.7 | 75 |
| 240 | 4.2 | 8.7 $\pm$ 0.05 | 10 $\pm$ 7 | 3.1 | 65 |
| 330 | 3.0 | 12.5 $\pm$ 0.16 | 12 $\pm$9 | 3.2 | 57 |

(continued)

| Volume of AgNO$_3$ sol. (50 mM) in the reaction medium ($\mu$l) | NaBH$_4$/ AgNO$_3$ molar ratio | AgNP content in the hybrid material (mg/g, wt%)[1] | Average Feret's diameter (nm)[2] | Crystallite size (nm)[3] | Ago (%)[4] |
|---|---|---|---|---|---|
| 550 | 1.8 | 18.6 $\pm$ 0.14 | 12.$\pm$ 9 | - | 32 |
| 700 | 1.5 | 24.7 $\pm$0.34 | 11 $\pm$ 9 | 3.0 | 34 |

Biocide activity - disk diffusion test

The short-term biocide activity at 48h of CNC/AgNP hybrids was monitored as a function of the AgNP content. The results, expressed as the size of the inhibition halo as a function of the weight content of silver in the sample (aqueous suspension of CNC/AgNP hybrid particles) in shown on figure 3. It can be observed that all the hybrid materials have a good biocidal activity (as a comparison, an aqueous suspension of pure CNC was also tested, and did not display any biocide activity).

The minimum inhibitory concentration (MIC) of the hybrid materials was estimated considering the first detectable inhibition halo which corresponded to the CNC/AgNP hybrid at AgNP content of 0.016 mg_AgNP/g_sample. Since only 3 $\mu$L of hybrid suspension were used for the test, it results that the minimum AgNP amount to obtain an antibacterial effect on the *Bacillus subtilis* strain is equal to 0.048 $\mu$g (i.e., 16 $\mu$gAgNP/mL).

This is the lowest AgNP content allowing to obtain a well-detectable biocide effect, compared to the materials based on silver nanoparticles proposed by the prior art.

Biocide activity - persistence test

Images of the inhibition halos obtained for the hybrid materials at 8.7 wt% and 24.7 wt% AgNP, after different incubation times (i.e., 48 h, 96 h, 120 h and 168 h) are shown in figure 4. Similar results are obtained for the other hybrid materials prepared according to the invention.

After 96 h, when the Ag$^+$ release seemed to reach an equilibrium state for both samples, the biocide-impregnated paper disks were layered on a fresh bacteria plate, rehydrated with 3 $\mu$L of H$_2$O and incubated until 120 h and 168 h. An anti-bacterial activity was still observed for both samples. This experimental evidence clearly shows that the 10 nm nano-spherical shaped silver nanoparticles of the hybrid materials prepared according to the invention have a highly efficient long-term biocide effect.

These results show that a particularly high biocidal activity can be obtained by a method of preparing hybrid materials based on silver nanoparticles grafted on cellulose nanocrystals, comprising very few steps, using unmodified cellulose nanocrystals as a starting material and a low amount of silver, without needing to perform any preliminary surface-modification of cellulose nanocrystals or any post-treatment step on the hybrid sample.

**EXAMPLE 2** - Chitin

Chitin nanocrystals (ChiNCs) were obtained by acid hydrolysis of shrimp shells purchased from Sigma-Aldrich. At the end of the synthesis, the ChiNCs were dispersed in an H$_2$O / HCl solution at pH 5.5 to prevent aggregation of the ChiNCs. The average size of a chitin nanocrystal is: 250 nm in length, 50 nm in width, 5 nm in thickness.

For the preparation of the hybrid particles made of ChiNCs and silver nanoparticles by a method according to the invention, the protocol as in Example 1 was used, with the following ratios:

- AgNO$_3$ / ChiNC mass ratio: 0.12 (g AgNO$_3$/g CNC);

- NaBH$_4$ / AgNO$_3$ molar ratio: 1.5 (nNaBH$_4$/nAgNO$_3$);

- theoretical AgNP level in the hybrid particles of 33.7 wt% (mg Ag/g hybrid particles).

The real level of AgNP in the hybrid material obtained at the end of the method of the invention was 23.5 wt%, as measured by atomic absorption spectroscopy.

The hybrid material obtained was characterized by different techniques, according to the characterization protocols described in Example 1.

Figure 5 shows a STEM image of this material. It can be observed that silver nanoparticles are well dispersed and grafted on the surface of the ChiNCs, as in the case of CNCs described in Example 1. Analysis of these images made it possible to establish the average size of the AgNPs around 16 ± 10 nm. The good dispersion of AgNPs of this size is also proven by the presence of the characteristic peak at λ = 400 nm in the UV-Vis spectrum. Finally, the XRD diffractograms, shown in figure 6, show an fcc (face centered cubic) structure for AgNPs. All these results agree with those found for the CNC / AgNP hybrid material according to the invention.

**Claims**

1. Method for preparing a hybrid material in particulate form, containing polysaccharide nanocrystals and silver nanoparticles, **characterized in that** it comprises:

   - a step of preparing an aqueous reaction medium containing native unmodified polysaccharide nanocrystals, a silver salt and a silver ion-reducing agent, and wherein the molar ratio "reducing agent / silver salt" is between 1 and 2000 and the mass ratio "silver salt / polysaccharide nanocrystals" is between 0.0002 and 0.5,
   - and a reaction step of allowing said reaction medium sufficient time to obtain therein hybrid particles comprising said polysaccharide nanocrystals on the surface of which are grafted silver nanoparticles.

2. Method according to claim 1, which is devoid of treatment step after said reaction step.

3. Method according to any of claims 1 or 2, wherein the molar ratio "reducing agent / silver salt" in said reaction medium is between 1.5 and 68.

4. Method according to any of claims 1 to 3, wherein the mass ratio "silver salt / polysaccharide nanocrystals" in said reaction medium is between 0.006 and 0.3.

5. Method according to any of claims 1 to 4, wherein said polysaccharide is chosen from cellulose, chitin, starch, mannan and any of the mixtures thereof.

6. Method according to any of claims 1 to 5, wherein said reducing agent is chosen from sodium borohydride, hydrazine, dimethylformamide, ammonia, ascorbic acid and reducing agents of plant origin.

7. Method according to any of claims 1 to 6, wherein said silver salt is silver nitrate or silver chloride.

8. Method according to any of claims 1 to 7, wherein the concentration of said silver salt in said reaction medium is between 0.005 and 1 % by weight of silver salt with respect to the total volume of said reaction medium.

9. Method according to any of claims 1 to 8, wherein said reaction step is carried out for at least 10 minutes, preferably between 1 and 24 hours.

10. Method according to any of claims 1 to 9, wherein said step of preparing said aqueous reaction medium comprises the successive steps of mixing an aqueous suspension of said polysaccharide nanocrystals with said silver salt, then, after one minute of stirring, adding said reducing agent.

11. Method according to any of claims 1 to 10, comprising a final step of purifying the hybrid particles obtained at the end of said reaction step.

12. Hybrid material obtainable by a method according to any of claims 1 to 11, **characterized in that** it consists of hybrid particles comprising unmodified polysaccharide nanocrystals on the surface of which are grafted silver nanoparticles, said silver nanoparticles having a spherical shape and a diameter of between 3 and 20 nm, and said hybrid particles containing 0.1 to 40 % by weight of silver, with respect to the total weight of said hybrid particles.

13. Hybrid material according to claim 12, having a minimal inhibiting concentration (MIC) against *Bacillus subtilis,* expressed with respect to silver nanoparticles contained in said hybrid particles, of 0.16 μg/ml.

14. Biocide composition comprising a hybrid material according to claim 12 or 13 in an aqueous vehicle.

15. Non-therapeutic use of a hybrid material according to claim 12 or 13 or of a biocide composition according to claim 14, as biocide agent, in particular for the protection of surfaces or in packaging for food products or cosmetic products.

FIG 1

FIG 2

EP 3 925 447 A1

FIG 3

FIG 4

14

FIG 5

FIG 6

Europäisches
Patentamt

European
Patent Office

Office européen
des brevets

# EUROPEAN SEARCH REPORT

Application Number

EP 20 30 5658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SINGLA RUBBEL ET AL: "In situfunctionalized nanobiocomposites dressings of bamboo cellulose nanocrystals and silver nanoparticles for accelerated wound healing", CARBOHYDRATE POLYMERS, APPLIED SCIENCE PUBLISHERS, LTD. BARKING, GB, vol. 155, 21 August 2016 (2016-08-21), pages 152-162, XP029756473, ISSN: 0144-8617, DOI: 10.1016/J.CARBPOL.2016.08.065 * paragraphs [02.2], [03.3]; figures 1 (c, d, e, f); table 2 * | 1-15 | INV.<br>A01N59/16<br>A01N25/10<br>A61K9/16<br>A61K33/38<br>A61L2/238<br>A01P1/00<br>A61K47/36<br>B22F9/24<br>B82Y40/00 |
| X | ARCOT R. LOKANATHAN ET AL: "Cellulose Nanocrystal-Mediated Synthesis of Silver Nanoparticles: Role of Sulfate Groups in Nucleation Phenomena", BIOMACROMOLECULES, vol. 15, no. 1, 17 December 2013 (2013-12-17), pages 373-379, XP55746194, ISSN: 1525-7797, DOI: 10.1021/bm401613h * "Synthesis of..."; page 374, column 2; figures 5, 7 * | 1-15 | |
| X | LIU KAI ET AL: "Preparation of the CNC/Ag/beeswax composites for enhancing antibacterial and water resistance properties of paper", CARBOHYDRATE POLYMERS, APPLIED SCIENCE PUBLISHERS, LTD. BARKING, GB, vol. 142, 22 January 2016 (2016-01-22), pages 183-188, XP029433066, ISSN: 0144-8617, DOI: 10.1016/J.CARBPOL.2016.01.044 * paragraph [2.2.1]; figures 2, 5 * | 1-15 | TECHNICAL FIELDS SEARCHED (IPC)<br><br>A61L<br>A61K<br>A01N<br>C22C<br>B82Y<br>B22F |

-/--

The present search report has been drawn up for all claims

| Place of search | Date of completion of the search | Examiner |
|---|---|---|
| Munich | 4 November 2020 | Sawicki, Marcin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 30 5658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XU XU ET AL: "Properties of novel polyvinyl alcohol/cellulose nanocrystals/silver nanoparticles blend membranes", CARBOHYDRATE POLYMERS, APPLIED SCIENCE PUBLISHERS, LTD. BARKING, GB, vol. 98, no. 2, 7 August 2013 (2013-08-07), pages 1573-1577, XP028718922, ISSN: 0144-8617, DOI: 10.1016/J.CARBPOL.2013.07.065 * paragraph [02.3]; figure 2b; table 1 * | 1-15 | |
| X | US 2011/262646 A1 (STANCIU LIA ANTOANETA [US] ET AL) 27 October 2011 (2011-10-27) * paragraphs [0054], [0056], [0065], [0083]; figure 7 * | 1-15 | |
| X | CN 111 250 728 A (WUXI DK ELECTRONIC MAT INC) 9 June 2020 (2020-06-09) * paragraph [0017] * | 1-14 | |

TECHNICAL FIELDS
SEARCHED      (IPC)

The present search report has been drawn up for all claims

| Place of search | Date of completion of the search | Examiner |
|---|---|---|
| Munich | 4 November 2020 | Sawicki, Marcin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

EP 3 925 447 A1

## ANNEX TO THE EUROPEAN SEARCH REPORT
## ON EUROPEAN PATENT APPLICATION NO.

EP 20 30 5658

This annex lists the patent family members relating to the patent documents cited in the above-mentioned European search report.
The members are as contained in the European Patent Office EDP file on
The European Patent Office is in no way liable for these particulars which are merely given for the purpose of information.

04-11-2020

| Patent document cited in search report | | Publication date | Patent family member(s) | Publication date |
|---|---|---|---|---|
| US 2011262646 | A1 | 27-10-2011 | NONE | |
| CN 111250728 | A | 09-06-2020 | NONE | |

EPO FORM P0459

For more details about this annex : see Official Journal of the European Patent Office, No. 12/82

18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **UDDIN et al.** *Green Mater.,* 2014, vol. 2, 183-192
- **SHI et al.** *J. Mater. Chem. B,* 2015
- **DROGAT et al.** *J. Nanoparticle Res.,* 2011, vol. 13, 1557-1562
- **MOON et al.** *Chem. Soc. Rev.,* 2011, vol. 40, 3941-3994
- **REID et al.** *Langmuir,* 2017, vol. 33 (7), 1583-1598